# Europäisches Patentamt

## European Patent Office

## Office européen des brevets

(11) Veröffentlichungsnummer: **0 143 218**

**B1**

(12) **EUROPÄISCHE PATENTSCHRIFT**

(45) Veröffentlichungstag der Patentschrift:
06.04.88

(21) Anmeldenummer: **84110553.9**

(22) Anmeldetag: **05.09.84**

(51) Int. Cl.⁴: **C 07 C 149/247**

(54) Verfahren zur Hydrolyse von 5-(beta-Methylmercapto-ethyl)-hydantoin.

(30) Priorität: 29.09.83 DE 3335218

(43) Veröffentlichungstag der Anmeldung:
05.06.85 Patentblatt 85/23

(45) Bekanntmachung des Hinweises auf die Patenterteilung:
06.04.88 Patentblatt 88/14

(84) Benannte Vertragsstaaten:
BE DE FR IT

(56) Entgegenhaltungen:
US-A-4 272 631

CHEMICAL ABSTRACTS, Band 84, Nr. 7, 16 Februar
1976, Seite 556, Zusammenfassung Nr. 44666k,
Columbus, Ohio, US
CHEMICAL ABSTRACTS, Band 85, Nr. 7, 16. August
1976, Seite 577, Zusammenfassung Nr. 47049t,
Columbus, Ohio, US

(73) Patentinhaber: Degussa Aktiengesellschaft,
Weissfrauenstrasse 9, D-6000 Frankfurt am Main
1 (DE)

(72) Erfinder: Bolze, Rudolf, Dr. Dipl.- Chem.,
Grünaustrasse 11, D-6450 Hanau 9 (DE)
Erfinder: Geiger, Friedhelm, Dr. Dipl.- Chem., Kurt-
Schumacher- Strasse 30a, D-6451 Erlensee (DE)
Erfinder: Spindler, Manfred, Dr. Dipl.- Chem.,
Kurfürstenstrasse 24, D-6450 Hanau 1 (DE)
Erfinder: Tanner, Herbert, Dr. Dipl.- Chem.,
Wildaustrasse 20, D-6450 Hanau 9 (DE)

## Beschreibung

Die Erfindung betrifft ein Verfahren zur Herstellung einer von Fremdsalzen praktisch freien Alkalimetallmethioninat-Lösung durch Hydrolyse von 5 - (β -Methylmercaptoethyl)-hydantoin in Gegenwart mindestens eines Alkalimetallhydroxids, -carbonats und/oder -hydrogencarbonats und von überschüssigem Ammoniak bei einer Temperatur zwischen 130 und 190°C und dem sich bei der gewählten Temperatur einstellenden Eigendruck.

Aus JP-A-75 106 901, referiert in Chem. Abs. Band 84 (1976), Seiten 556-557, Referat Nr. 44666k, ist es bekannt, Methionin durch Hydrolyse von 5-(β -Methylmercaptoethyl)-hydantoin in Gegenwart von etwa 1,2 Äquivalenten Natriumhydroxid und von etwa 9 Äquivalenten Ammoniak bei 180°C herzustellen. Die bei dieser Arbeitsweise intermediär entstehende Natriummethioninat-Lösung enthält jedoch zwangsläufig neben dem Natriummethioninat auch Natriumcarbonat.

Das erfindungsgemäße Verfahren ist dadurch gekennzeichnet, daß man, jeweils bezogen auf die Hydantoinmenge, die Alkalimetallverbindung in 1 Äquivalent und den Ammoniak in einem Überschuß von 1 bis 15 Äquivalenten einsetzt und während der Hydrolyse die entstehenden gasförmigen Verbindungen ganz oder teilweise aus dem Reaktionssystem abtrennt und gleichzeitig den überschüssigen Ammoniak zusetzt.

Der Ammoniak wird dem Reaktionssystem während des Verlaufs der Hydrolyse kontinuierlich oder diskontinuierlich in Gasform und/oder als wäßrige, vorzugsweise konzentrierte Ammoniumhydroxidlösung zugesetzt. "Überschüssiger Ammoniak" bedeutet: Zusätzlich zu dem während der Hydrolyse entstehenden Ammoniak.

Die Verwendung von nur 1 Äquivalent der Alkalimetallverbindung macht das erfindungsgemäße Verfahren insofern besonders vorteilhaft, weil man nach dem an sich bekannten Austreiben des Ammoniaks und des Kohlendioxids aus dem nach Abschluß der Hydrolyse vorliegenden Reaktionsgemisch eine Alkalimetallmethioninat-Lösung erhält, die praktisch frei von Fremdsalzen und daher besonders geeignet zur Supplementierung von Tierfutter mit Methionin ist.

Aus dieser Lösung kann auch in vorteilhafter Weise durch Neutralisation mit einer Säure nach dem Stand der Technik Methionin ausgefällt und isoliert werden. Dadei müssen erheblich geringere Mengen an Fremdsalz abgetrennt werden als nach den bisher bekannten Verfahren, und die Belastung der Abwässer wird entsprechend verringert.

Vorzugsweise eingesetzt werden folgende Alkalimetallverbindungen: Natriumhydroxid, Kaliumhydroxid, Natriumhydrogencarbonat, Kaliumhydrogencarbonat, Natriumcarbonat und Kaliumcarbonat.

In einer geeigneten Ausführungsform führt man die Hydrolyse z. B. in einem Autoklaven unter dem sich jeweils einstellenden Eigendruck durch, der sich hauptsächlich aus den Partialdrucken des Wasserdampfs, des bei der Reaktion entstehenden Kohlendioxids und Ammoniaks, sowie des überschüssigen Ammoniaks zusammensetzt, soweit diese Gase nicht in dem Reaktionsgemisch gelöst sind. Für die kontinuierliche Durchführung des Verfahrens eignen sich z. B. druckfeste Kolonnen.

Bei der kontinuierlichen Verfahrensweise wird das Reaktionsgemisch während einer Zeitdauer durch eine Reaktionszone geführt, die ausreicht, das Hydantoin zu hydrolysieren.

Das erfindungsgemäße Verfahren wird zweckmäßigerweise so durchgeführt, daß man die während der Hydrolyse entstehenden gasförmigen Verbindungen Kohlendioxid und Ammoniak kontinuierlich oder diskontinuierlich, bevorzugt kontinuierlich, aus dem Gasraum über dem Reaktionsgemisch abzieht und gleichzeitig auch den dabei im Reaktionssystem auftretenden Ammoniakverlust durch Zusatz entsprechender Mengen Ammoniak in Gasform oder als Ammoniumhydroxidlösung ausgleicht. Dadurch wird die für die erfolgreiche Durchführung des erfindungsgemäßen Verfahrens geltende Voraussetzung erfüllt, daß während der Hydrolyse ein Ammoniaküberschuß einwirken muß.

Die während bzw. nach Beendigung der Hydrolyse abgezogenen Gase Kohlendioxid und Ammoniak können nach destillativer Aufarbeitung recycliert werden. Das verfahrengsgemäß eingesetzte Hydantoin muß keine reine Verbindung sein. Beispielsweise kann man ein hydantoinhaltiges Gemisch einsetzen, welches man durch Umsetzung von 3-Methylmercaptopropionaldehyd, Ammoniumbicarbonat und einer Cyanidverbindung in wässriger Lösung nach dem Stand der Technik hergestellt hat.

Die für die spätere Verwendung gewünschte Konzentration an Alkalimetallmethioninat kann entweder durch entsprechende Auswahl der Hydantoin -Konzentration oder durch Verdünnen oder Einengen der nach der Hydrolyse anfallenden Lösung eingestellt werden.

Das erfindungsgemäße Verfahren wird durch das nachfolgende Beispiel näher erläutert:

### Beispiel:

Auf den Kopf einer druckfesten Kolonne werden stündlich eine nach üblichem Verfahren hergestellte Lösung von 174,2 kg (1 kMol) 5-(β -Methylmercaptoethyl)-hydantoin in 260 kg Wasser sowie 80 kg 50 %-iger (= 1 kMol) Natronlauge gegeben. Gleichzeitig leitet man in den unteren Teil der Kolonne 34,1 kg/h (2 kMol) gasförmiges Ammoniak ein und stellt eine Reaktionstemperatur von 160°C ein. Am Kopf der

2

Kolonne werden Ammoniak, Kohlendioxid und Wasserdampf über ein Druckhalteventil abgezogen. Das den Sumpf der Kolonne verlassende Hydrolysat wird entspannt und abgekühlt. Die Ausbeute an Methionin beträgt 97,0 %.

Wird die kontinuierliche Verseifung unter sonst gleichen Bedingungen, jedoch ohne Einleiten von Ammoniak durchgeführt, dann entsteht Methionin in 81,2 % Ausbeute.

**Patentansprüche**

1. Verfahren zur Herstellung einer von Fremdsalzen praktisch freien Alkalimetallmethioninat-Lösung durch Hydrolyse von 5-(β -Methylmercaptoethyl)-hydantoin in Gegenwart mindestens eines Alkalimetallhydroxids,-carbonats und/oder -hydrogencarbonats und von überschüssigem Ammoniak bei einer Temperatur zwischen 130 und 190°C und dem sich bei der gewählten Temperatur einstellenden Eigendruck, dadurch gekennzeichnet, daß man, jeweils bezogen auf die Hydantoinmenge, die Alkalimetallverbindung in 1 Äquivalent und den Ammoniak in einem Überschuß von 1 bis 15 Äquivalenten einsetzt und während der Hydrolyse die entstehenden gasförmigen Verbindungen ganz oder teilweise aus dem Reaktionssystem abtrennt und gleichzeitig den überschüssigen Ammoniak zusetzt.

2. Verfahren nach Anspruch 1, dadurch gekennzeichnet, daß man den Ammoniak in Gasform und/oder als wäßrige Ammoniumhydroxidlösung zusetzt.

3. Verfahren nach Anspruch 1 oder 2, dadurch gekennzeichnet, daß man die Abtrennung der entstehenden gasförmigen Verbindungen aus dem Reaktionssystem kontinuierlich oder diskontinuierlich vornimmt und den Ammoniak in entsprechender Weise zusetzt.

4. Verfahren nach einem der Ansprüche 1 bis 3, dadurch gekennzeichnet, daß man nach Abschluß der Hydrolyse den restlichen Ammoniak und das restliche Kohlendioxid aus dem Hydrolysat abtrennt.

**Claims**

1. A process for the preparation of an alkali metal methioninate solution substantially free from foreign salts by hydrolysis of 5-(β-methylmercaptoethyl)-hydantoin in the presence of at least one alkali metal hydroxide, carbonate and/or hydrogen carbonate and excess ammonia at a temperature of from 130 to 190°C and under the pressure spontaneously established at the temperature selected, characterized in that, based in each case on the quantity of hydantoin, the alkali metal compound is used in 1 equivalent and the ammonia in an excess of from 1 to 15 equivalents and in that the gaseous compounds formed are completely or partly removed from the reaction system during the hydrolysis and, at the same time, the excess ammonia is added.

2. A process as claimed in Claim 1, characterized in that the ammonia is added in gaseous form and/or as an aqueous ammonium hydroxide solution.

3. A process as claimed in Claim 1 or 2, characterized in that the gaseous compounds formed are removed from the reaction system continuously or at intervals and the ammonia correspondingly added.

4. A process as claimed in any of Claims 1 to 3, characterized in that, on completion of hydrolysis, the residual ammonia and the residual carbon dioxide are removed from the hydrolyzate.

**Revendications**

1. Procédé pour la fabrication d'une solution, pratiquement exempte de sels étrangers, de méthioninate de métal alcalin, par hydrolyse de 5-(β -méthylmercaptoéthyl)-hydantoïne, en présence d'au moins un hydroxyde, carbonate, et/ou bicarbonate de métal alcalin, et d'un excès de l'ammoniac à une température entre 130 et 190°C, et de la pression qui s'établit à la température choisie, procédé caractérisé en ce que l'on met en oeuvre, en calculant toujours sur la quantité d'hydantoïne, le composé de métal alcalin à raison d'un équivalent, et l'ammoniac en un excès de 1 à 15 équivalents, et pendant l'hydrolyse, on sépare du système réactionnel, totalement ou partiellement les composés gazeux qui se sont formés, et ajoute en même temps l'excès de gaz ammoniac.

2. Procédé suivant la revendication 1, caractérisé en ce que l'on ajoute l'ammoniac sous la forme de gaz et/ou sous la forme d'une solution aqueuse d'hydroxyde d'ammonium.

3. Procédé suivant les revendications 1 ou 2, caractérisé en ce que l'on procède à la séparation des composés gazeux, qui se sont formés, du système réactionnel d'une façon continue ou discontinue, et ajoute l'ammoniac dans des conditions correspondantes.

4. Procédé suivant l'une des revendications 1 à 3, caractérisé en ce que, l'hydrolyse terminée, on sépare de l'hydrolysat le reste de l'ammoniac et le reste du dioxyde de carbone.